# EUROPEAN PATENT APPLICATION

(11) **EP 4 684 675 A1**
(43) Date of publication of application: **28.01.2026**
(21) Application number: 24191025.6
(22) Date of filing: 25.07.2024
(51) Int. Cl.: A43B 3/34, A43B 17/00, A61N 1/40, A61N 7/00, A61N 2/02

(54) **THERAPEUTIC SYSTEM IN THE FORM OF FOOTWEAR DEDICATED TO PATIENTS WITH DIABETIC FOOT SYNDROME**

(30) Priority: 22.07.2024 PL 44928724
(71) Applicant: Siec Badawcza Lukasiewicz Lodzki Instytut Technologiczny, 90-570 Lodz (PL); Siwak, Mateusz, 90-419 Lodz (PL); Majsterek, Ireneusz, 92-413 Lodz (PL)
(72) Inventor: Siwak, Mateusz, Lodz (PL); Majsterek, Ireneusz, Lodz (PL); Lawinska, Katarzyna, Rabien AB (PL); Gajewski, Robert, Krakow (PL); Maslowska-Lipowicz, Iwona, Lodz (PL)
(74) Representative: Wroblewski, Michal

(57) **Abstract**

A therapeutic system in the form of footwear dedicated to patients with diabetic foot syndrome, characterised by all the features and ability to provide active therapy to patients, primarily in the neurogenic form of the disease. The system takes the form of a device comprising shoes comprising therapeutic plantar insoles (1) with ultrasound emitters (3a, 3b) and magnetic field emitters (2) and a portable device for electrical stimulation in the form of a controller (5).

## Description

### FIELD

The object of the invention is a therapeutic system in the form of footwear dedicated to patients with diabetic foot syndrome, characterised by all the features and ability to provide active therapy to patients, primarily in the neurogenic form of the disease.

Diabetic foot syndrome is a serious health problem that requires a multidirectional therapeutic approach. Both prevention and appropriate treatment of wounds that may occur as a result of neuropathy or ischaemia play an important role. The lifetime risk of developing an ulcer in a diabetic patient ranges from 12 to as much as 25%, and patients with diabetes are as much as 30 to 40 times more likely to have an amputation than those without diabetes. A high-risk foot is a foot at risk of developing an ulcer in a short period of time. Muscle atrophy, hammer toes, and calluses are typical for this type of foot. If additional factors are present, such as poor foot hygiene, ill-fitting footwear and mechanical, thermal or chemical trauma, ulceration will occur. An ulceration is considered to be either a shallow or deep break in skin continuity, usually located on the plantar or dorsal part of the foot. It is most often found under the heads of the metatarsal bones, on the dorsum of the toes or on the heel.

Skin wounds and ulcers that develop as a result of diseases such as diabetes or mechanical bedsores can be very painful and usually seriously reduce the patient's quality of life. Furthermore, skin ulcers occurring in the course of metabolic diseases such as diabetes are chronic and usually difficult to treat. In many cases, the body, despite intensive treatment with the methods used for the time being, is unable to regenerate effectively, often leading to amputation. Ultrasound and magnetic field therapy are well established and documented in the treatment of chronic wounds. For wounds associated with diabetic foot syndrome, pulsed ultrasound therapy is an ideal way to accelerate wound healing, particularly for connective tissue wounds. The mechanism of action is pleiotropic, with the observed and described mechanisms including increased blood flow and stimulation of collagen production, which accelerates regenerative processes. The pulsed magnetic field has a documented anti-oedema effect, suppresses ongoing inflammation, improves microcirculation, the cumulative effect of which contributes to faster wound healing. Numerous reports indicate that pulsed magnetic field therapy can also reduce the pain and oedema associated with diabetic foot syndrome.

### STATE OF THE ART

Systems of this type are known in many embodiments.

International application WO2011094464A1 discloses a system configured to be worn by the user and provide electrical stimulation to the user. The system comprises: a power source configured to generate power at least partially using pressure or displacement resulting from the normal movements of said user and at least one conductive wire connected to the power source, configured to transmit electrical stimulation from said power source to a predetermined area of the user. At least one conductive wire contains an anode and a cathode configured to form a complete circuit when the user is in contact with said system. The power source is a piezoelectric cell. The piezoelectric cell is circular in shape and approximately 10 to 40 mm in diameter. The piezoelectric cell has a voltage in the approximate range of at least 2.5 - 250 V and generates a current in the approximate range of 25 - 500 µA. The power source includes at least two piezoelectric cells in series. The power source is located between the metatarsal area and the user's heel area. The shoe includes an insole formed at least partially of a moulded gel and foam rubber, an interior formed at least partially of a melted gel orfoam rubber, and wherein the at least one conductive wire is formed of a material consisting of a silver-and carbon-coated nylon thread, carbon nanofibre threads.

Patent specification US10326312B2 discloses a mobile terminal charging apparatus comprising an energy-harvesting device placed in shoes and producing energy generated by the movement of the human body, a charging control module attached to the trousers and a module controlling the charging of the mobile terminal using the energy harvested by the energy-harvesting device, and a resonant coil module wirelessly transmitting the energy harvested by the energy-harvesting device to the charging control module. The resonant coil module comprises a transmitting resonant coil placed in the shoes and a receiving resonant coil placed in the trousers, located at a certain distance from the transmitting resonant coil and magnetically connected to the transmitting resonant coil. The transmitting resonant coil assembly is wound along the circumference of the upper shoe line and the receiving resonant coil assembly along the circumference of the trouser cuffs. The energy-harvesting device is at least one piezoelectric device that converts the pressure applied by the movement of the human body into energy.

Patent US7424325B2 discloses a piezoelectrically stimulated device for utilising an electrical potential on the skin surface of the human body comprising: a piezoelectric element capable of generating an electrical potential in response to mechanical stress or deformation of said piezoelectric element caused by movement of the human body, with electrodes capable of contacting the skin surface of the human body; an electrical circuit connected to said piezoelectric element for the extraction, storage or modification of said electrical potential, connected to at least two electrodes of opposite polarity, for the use of said electrical potential on the surface of the skin of the human body for transdermal drug delivery, treatment of pathologies, wounds or injuries, transient nerve stimulation for pain relief and other applications. The model electrical circuit of the device comprises a piezoelectric element connected to a rectifier bridge, a resistor and a voltmeter. The piezoelectric element consists of a bimorph zirconium titanate fibre.

Utility model JP1993091404U discloses a device in which a piezoelectric element is positioned in the heel area of the footwear and an electromagnetic coil is positioned in the front area, wherein the piezoelectric plate is a passive element that generates power for the electromagnetic coil, which is the actual generator of the electromagnetic field. The described solution reveals the use of a piezoelectric element as a direct power source. There is no information on the possibility of passively using a piezoelectric element to charge an energy source such as a battery. Furthermore, there is no component controlling the operation of the sub-assembly, forcing it to operate according to preset frequencies that are optimal from a therapeutic point of view. The solution also has a separate arrangement of the piezoelectric part and the solenoid. Most importantly, the disclosed solution does not provide ultrasound therapy, and certainly not a controlled and coordinated adjuvant therapy involving the resultant interaction of pulsed ultrasound and a pulsed eletromagnetic field.

### ESSENCE OF THE INVENTION

The essence of the solution according to the invention is a therapeutic system in the form of footwear dedicated to patients with diabetic foot syndrome, meeting the ergonomic standards specific to the certificate "Footwear for diabetics", where it is particularly important to provide shock absorption, a sufficiently spacious interior, allowing the foot to be positioned freely in a way that prevents pressure on the toes and other sensitive areas, the minimisation or complete absence of seams inside the shoe, in order to avoid abrasion and irritation of the skin and to provide adjustable fasteners in the form of, for example, Velcro or laces, allowing the adjustment of the footwear to the individual needs of the patient, providing a better fit, with the materials used to make the shoes being breathable and moisture-wicking to prevent excessive foot perspiration and the development of infections. Of no less importance is the shape that supports the arch of the foot, reducing stress on the joints and improving overall comfort of use.

The system comprises shoes with therapeutic plantar insoles, in detachable form or as an integral part of the shoe, adapted to provide electromagnetic stimulation by a pulsed electromagnetic field and mechanical stimulation by pulsed ultrasound, through pulsed electromagnetic field emitters and pulsed ultrasound emitters installed in the insoles. The emitters are connected by electrical wires to a portable, battery-powered power supply device in the form of a controller, which enables the therapeutic process to be carried out with adjuvant therapy of pulsed electromagnetic fields and pulsed ultrasound according to a preset programme of the professionally active patient's feet, during his or her daily activities. The emitters in the plantar insoles are installed vertically in the middle layer of the insole, and horizontally in the location of the most common ulceration in diabetic foot syndrome, i.e. in the heel area and between the heads of the first and fifth metatarsal bones. These include emitters that emit pulsed electromagnetic fields and emitters that emit pulsed ultrasound. Pulsed ultrasound emitters are located both within and between pulsed electromagnetic field emitters.

Coils are used as the pulsed electromagnetic field emitters.

Piezoelectric emitters selected from a group including rectangular, disk or ring-shaped ceramic piezoelectrics, sandwich-type batteries made of silver-plated PVDF piezoelectric film, polymer piezoelectric materials are used as the pulsed ultrasonic emitters. Preferably, ceramic disks are used as piezoelectric emitters.

Pulsed electromagnetic field emitters and pulsed ultrasound emitters are connected in parallel.

In the controller, there is a microcontroller module with integrated algorithms to operate the controller installation and an integrated display module, a voltage stabiliser module to provide the required supply voltage for the individual components, a voltage measurement module, a circuit temperature control module, a USB connector module with a converter, which allows communication between the microcontroller and a computer with a USB interface, a power supply and battery charging module configured to provide electrical stimulation that is above, at or below the threshold of human sensation and modules to control pulsed electromagnetic field emitters and ultrasound emitters. As the power module, lithium-ion batteries are used, connected in series to form packs, which are in turn connected in parallel to form a 2S2P configuration. The batteries are equipped with a BMS (Battery Management System) and voltage stabilisers.

The controller housing is in the form of a multi-walled casing, enclosed by a front cover with a set of four mounting holes by means of which the front cover is fixed to the housing. The front cover has a hole for the display and buttons used for user interaction with the device. In the rear wall of the housing, there are catches with retainers, allowing the casing to be mounted on a belt that passes through these retainers. In the front wall, there is a hole for a charging connector, a hole for a USB programming connector, a hole for a battery power connector, and in the side wall, there is a hole for a connector to connect therapeutic insoles.

The invention also relates to a method of electrical stimulation of the diabetic foot by implementing a therapeutic system, capable of generating an electrical potential in response to the straining of piezoelectric emitters caused by the movements of the human body, comprising therapeutic plantar insoles with pulsed electromagnetic field emitters and pulsed ultrasound emitters, connected by an electrical wiring assembly to a portable controller configured to deliver electrical stimulation to the patient's feet, characterised in that it comprises the steps of:
- selecting a therapeutic programme to achieve the desired effect,
- checking, for the selected therapeutic programme, the information provided by the system, which should be taken into account,
- administering electrical stimulation to the patient via the portable controller,
- subjecting the patient to alternating stimulation with pulsed electromagnetic field and pulsed ultrasound with pauses according to the therapeutic programme used,
- adjusting the length of stimulation,
- stopping stimulation if necessary.

The aim of the invention is to develop a therapeutic system of electrical stimulation for treating the foot of a patient with diabetic foot syndrome.

The purpose of the invention is achieved by the independent claims. The dependent claims define preferred embodiments. An example is provided to facilitate understanding of the present solution according to the invention.

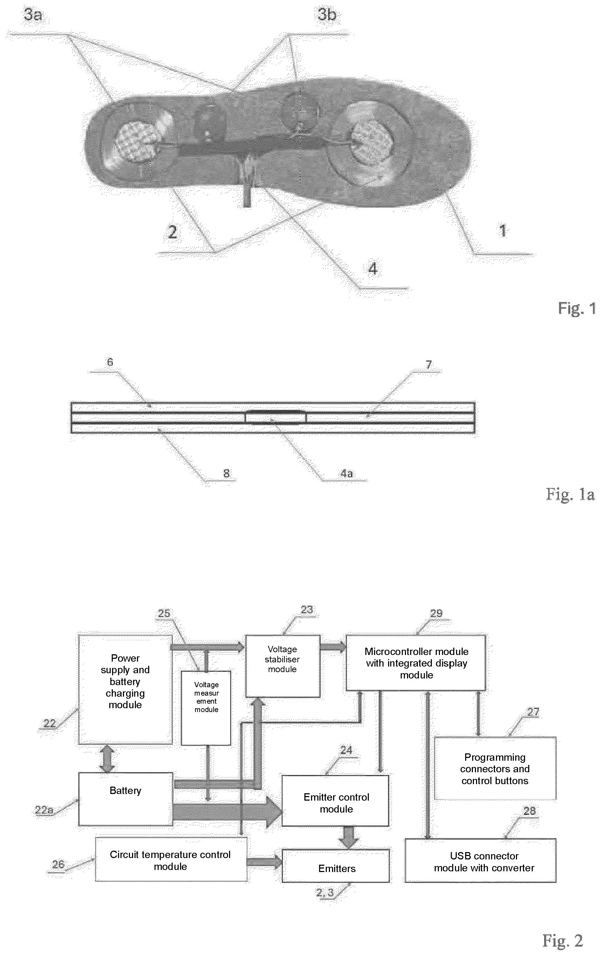

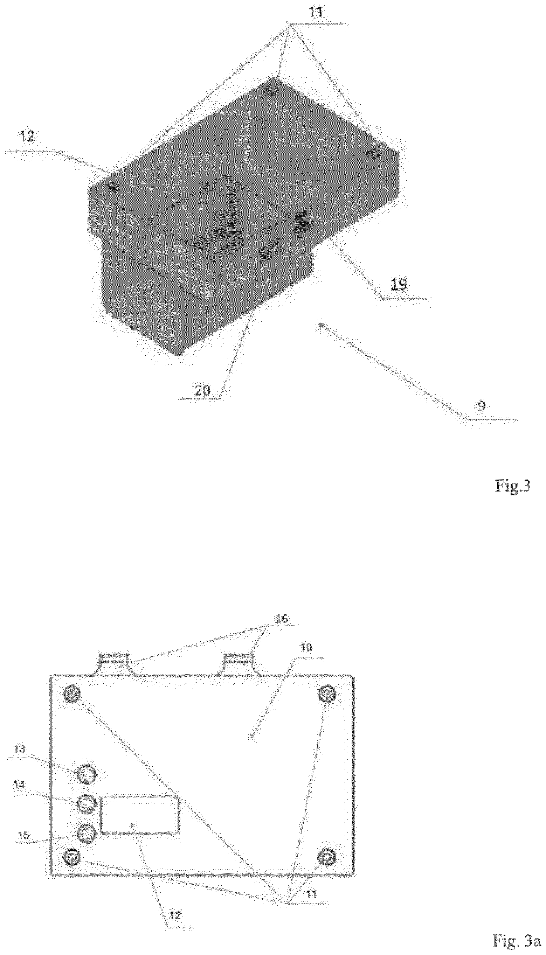

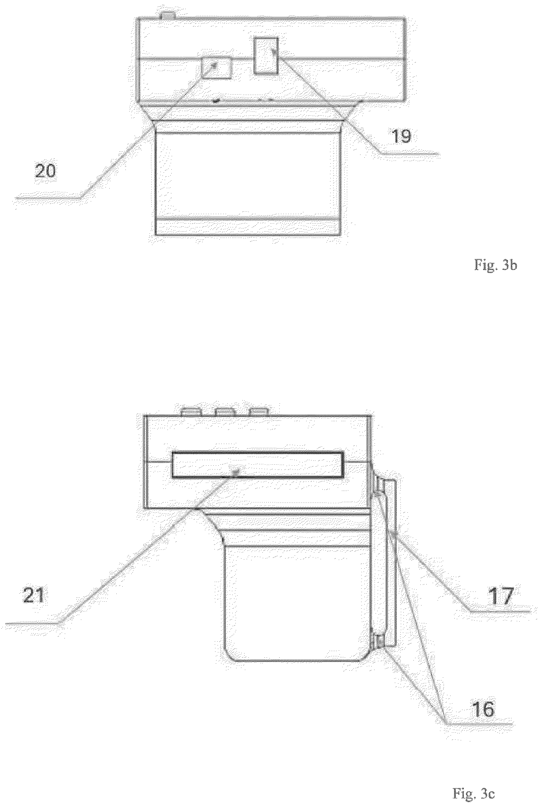

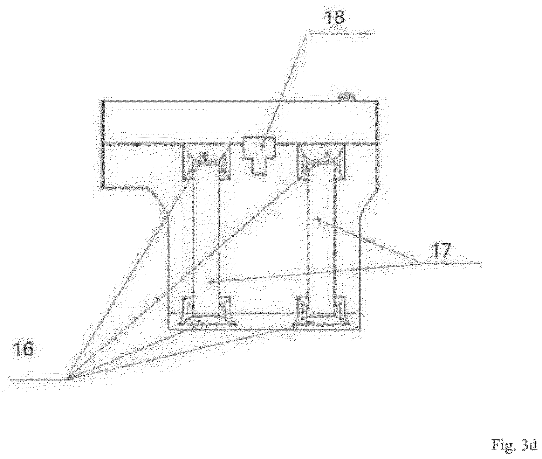

The object of the invention is shown in the embodiments and in the drawing, in which Fig. 1 shows a therapeutic plantar insole in a top view with the arrangement of the emitters, Fig. 1a - therapeutic plantar insole in a side view from the medial side, Fig. 2 - a block diagram of the controller showing an example of the system of individual modules, their schematic arrangement and interactions, Fig. 3 - a 3D view of the controller housing, Fig. 3a - a top view of the controller housing, Fig. 3b - a view of the controller housing from the right side, Fig. 3c - a view of the controller housing from the left side, Fig. 3d - a view of the controller housing from the back.

### Embodiment 1 of the device

An embodiment will be described in detail below with reference to the accompanying drawing figures. It should be appreciated that the following embodiment is not intended to limit the scope of the appended claims and that not all combinations of the features described in the example embodiment are necessarily relevant to the present invention. Of the many features described in the embodiment, two or more features can be freely combined.

The therapeutic system according to the invention takes the form of a device consisting of shoes comprising therapeutic plantar insoles 1 with pulsed electromagnetic field emitters 2 and pulsed ultrasound emitters 3a and 3b installed therein. The emitters are connected by electrical wires 4 to a portable device for electrical stimulation in the form of a controller 5.

Each plantar insole 1, viewed from the top, consists of the following three layers:
- layer 6 made of 3 mm thick foam with adhesive on the inside,
- layer 7 made of 7 mm thick thermal insole made of felt and foam,
- layer 8 of high-impact HIPS polystyrene, 1 mm thick,
where 4a denotes the wire output.

The entire device consists of two identical therapy insoles 1 - left and right.

The emitters are placed in the middle layer 7 in specially cut holes and are connected by electrical wires 4.

The pulsed electromagnetic field emitters are two coils 2, while the pulsed ultrasound emitters are four piezoelectric discs 3a and 3b, of which discs 3a are located inside coils 2 and discs 3b between coils 2.

Coils 2 are located under the areas where wounds most often develop in association with diabetic foot problems, that is, under the heel and under the forefoot. Each coil 2 is in the form of 350 turns wound from an enameled copper wire with a diameter of 0.425 mm. The inner diameter of the coil is 34 mm, the outer diameter is 68 mm, with a height of 5 mm.

Coils 2 according to the invention emit an electromagnetic field with an induction of not less than 5 mT at a distance of up to 2 cm above the coil location.

Piezoelectric ceramic discs 3a and 3b were used. Each disk has a nominal power of 10 W, which with four disks in the insole gives a total of 40 W of nominal power. Each of the discs 3a, 3b has the following dimensions: 28 mm x 2 mm. The static capacitance of the disk is 3700 pF, with a mass of 10 g.

Coils 2 and discs 3a, 3b in each shoe are connected by electrical wires 4 to a portable stimulating device in the form of a controller 5 installed in a housing 9 adapted to be worn on a waist belt or holster.

The housing 9 of the controller 5 is in the form of a multi-walled casing closed at the top by a front cover 10 with a set of four mounting holes 11 by which the front cover 10 is fixed to the casing. The front cover 10 has a hole 12 for a display and holes 13, 14, 15 for functional buttons that allow the user to interact with the device, allowing easy control of its functions. They can be used to start, stop measurements, reset the device or for other user-defined purposes.

In the rear wall of the housing 9, there are four catches 16 with two retainers 17, allowing the casing to be mounted on the belt that passes through these retainers, and a hole for a connector 18 for connecting the battery to a printed circuit board, PCB, not shown in the drawing.

The front wall of the housing 9 of the controller 5 has a hole 19 for the battery power connector and a hole 20 for the USB programming connector. In the side wall of the housing 9 of the controller 5, there is a hole 21 for a connector for connecting therapeutic insoles.

The electronic modules of the controller 5 are mounted on the PCB, not shown in the drawing, installed in the controller. The electronic modules and components provided on the PCB allow the portable device for electrical stimulation to be activated and controlled. These include a power supply and battery charging module 22, a battery 22a, a voltage stabiliser module 23, modules 24 for controlling individual emitters, a voltage measurement module 25, a circuit temperature control module 26, programming connectors with control buttons 27, a USB connector module with converter 28 and a microcontroller module integrated with a display module 29, able to power individual pulsed electromagnetic field emitters and pulsed ultrasound emitters housed in a therapeutic insole 1 or constituting a permanent structural element of the footwear sole, meeting the ergonomic requirements specific to the certificate "Footwear for diabetics".

Module 22 has the form of a 3500 mAh lithium-ion battery pack in a 2S2P configuration - two batteries 22a connected in series, and then two packs (2 pcs each) connected in parallel - which gives a total of 4 batteries in the entire pack. The 2S (2 Series) series configuration allows an increase in voltage, which is nominally 7.4 V. Lithium-ion batteries in a 2S configuration operate in the range from approx. 6 V to approx. 8.4 V. This range was adopted because of the pulsed electromagnetic field control, since a coil with many turns has a relatively high resistance and at low voltages there could be a problem with the current flowing through it. Another reason is that some integrated circuits are powered by 5 V, so it was decided to reduce the voltage by means of stabilisers. The 2P (2 Parallel) connection increases the capacity of the entire pack, and in this case it amounts to 7000 mAh (7 Ah). The battery pack is equipped with a BMS (Battery Management System), which protects against overcharging, discharging and handles the voltage balancing of the individual batteries.

The selected battery pack in the 2S2P configuration with a capacity of 7 Ah and a nominal voltage of 7.4 V should be more than sufficient to operate the device for the required 12 h with four therapy sessions of 20 minutes each. The batteries are charged with a charger with an output voltage of 8.4 V, a maximum current of 2 A and passively based on the voltage generated by the piezoelectrics when moving.

All electronic components on the PCB are in communication and synchronised with each other, working together to ensure reliable and efficient monitoring of therapy sessions. Their cooperation allows precise tracking of movements, analysis of data and provision of detailed information feedback to patients to help optimise therapy sessions.

On the PCB, in addition to advanced modules, there are also fundamental passive components such as resistors, capacitors and transistors. Despite their simplicity, they play a key role in ensuring that the entire system functions properly. Resistors help control the current in the circuit, capacitors store and release energy and transistors act as switches or signal amplifiers.

Their presence is essential to stabilise the system, protect against surges and ensure smooth and efficient communication between the various parts of the electronic system.

The method of operation of the active therapeutic footwear according to the invention is illustrated in the block diagram. The method consists in that, when the device powered in therapeutic mode from the dedicated battery 22a, previously charged via the power module 22, is switched on, the module 24 controlling the individual emitters is powered directly from the battery 22a and the microcontroller module 29 via a voltage stabiliser. The display presents the user with a choice of therapy programme(s), together with information on the dedicated therapy programme(s), and the battery charge level, assessed via the voltage measurement module 25. The user then selects, by means of the control buttons 27 connected to the microcontroller module 29, a dedicated or one of dedicated therapeutic programmes on the microcontroller module 29, previously programmed by means of the USB connector module 28, after which the current signal is transmitted to the control modules of the individual emitters, which, drawing the supply current from the battery 22a, supply the individual emitters according to the programmed algorithm executed within the therapeutic programme activated. The process is controlled in real time by measuring the voltage via the voltage measurement module 25 and controlling the temperature of the emitter assembly via the circuit temperature control module 26. This information is transmitted to the microcontroller module 29, which, if it detects values that do not comply with the accepted values, can stop the therapeutic process by giving an appropriate message via the display 29. Once the therapeutic programme is completed, the selection menu is returned.

The method of treating a patient with diabetic foot syndrome is carried out by alternately stimulating the patient with pulsed electromagnetic fields and pulsed ultrasound according to the therapeutic programme used, programmed on a portable controller containing a power module and a set of electric wires configured to deliver the adopted therapeutic agents, resulting in accelerated healing of wounds, particularly connective tissue wounds.

The activated device, based on an integrated algorithm operating the controller in an alternating manner, delivers a pulsed electromagnetic field and pulsed ultrasound with preset parameters. During operation of the device, the instantaneous value of the system temperature around the coils is continuously measured.

The operational parameters of the electrical stimulation (e.g. intensity, frequency, pulse duration) can change depending on the therapeutic programme used and during the therapy session itself, for example in response to clinical feedback and results.

The system is used to ensure that the patient can receive therapy over a 12-hour period, during which, for example, a 20-minute therapy series will occur four times over this period with a programme based on 20 seconds of ultrasound stimulation at 40 kHz, followed by 20 seconds of pulsed electromagnetic field stimulation at 50 Hz, followed by a 20-second pause. The data acquired from the emitters in the plantar insole, thanks to the integrated algorithms for operating the controller and the appropriate software, make it possible to activate and switch off the device, select a dedicated therapeutic programme, verify the battery level, read the number of therapeutic cycles completed in the last 24 hours with the help of the USB port on the controller and the display fitted, with an interface for acquiring data from the emitters in the plantar insole.

### ADVANTAGES

When working, the emitters emit energy which, thanks to the materials used, accumulates in the form of heat inside the insole and is dissipated throughout the therapy session over the entire surface of the insole. Such a solution proves useful for short sessions with long breaks. The coils do not heat above 37° C for a 20-minute therapeutic programme. It is possible to work safely in conditions 5 degrees higher.

The selected 2S2P battery pack, with a capacity of 7 Ah and a nominal voltage of 7.4 V, is sufficient to operate the device for the required 12 hours with four therapy sessions of 20 minutes each.

During a therapy session, it is possible to use the functionalities that have been implemented in the device:
- controlling the ultrasound emitter at 40 kHz separately for left and right insoles
- controlling the pulsed electromagnetic field emitter according to a preset frequency separately for the left and right insoles
- reading temperature values from the left and right insoles
- displaying a simple menu on the display
- reading the current battery status and displaying it on the screen
- controlling the device by means of buttons ( navigating through the menu, switching on/off)
- transmitting data when USB cable is connected
- possibility to program therapy sessions

The ergonomic design of the controller housing allows it to be worn on a dedicated waist belt or holster. It is possible to passively charge the batteries based on the voltage generated by piezoelectrics during movement.

Locating the wire connecting the plantar insoles to the controller in the middle part of the insole, on the inside, allows use of the natural space between the shoe and the foot, where there are no dynamic pressures that can cause painful poking of the wire into the foot, and allows reliable connection of the wire to the controller mounted to the trouser belt. The stresses on the wire, caused by the movement of the legs while walking, are minimal and are not transferred to the connector causing it to disengage itself. The system has a latch, not shown in the drawing, to prevent self-release.

## Claims

1. A therapeutic system in the form of footwear dedicated to patients with diabetic foot syndrome comprising shoes with multilayered therapeutic plantar insoles in detachable form or as an integral part of the footwear, pulsed electromagnetic field emitters and pulsed ultrasound emitters mounted in the insoles, connected by electrical wires to the electronic circuits of the controlle, **characterised in that** the emitters in the plantar insoles are mounted in a layer (7), with the pulsed ultrasound emitters (3a) being located inside the pulsed electromagnetic field emitters (2), and the pulsed ultrasound emitters (3b) between the emitters (2), wherein both the emitters (2) and the emitters (3a, 3b) are connected by electrical wires (4) to a portable device for electrical stimulation in the form of a controller (5), comprising a microcontroller module (29) with integrated algorithms for operating the controller installation and an integrated display module, a voltage stabiliser module (23), a voltage measurement module (25), a circuit temperature control module (26), a USB connector module with converter (28), a power and battery charging module (22) configured to provide electrical stimulation and modules (24) for controlling pulsed electromagnetic field emitters and ultrasound emitters.

2. The system according to claim 1, **characterised in that** the emitters (2) and the emitters (3a, 3b) are connected in parallel.

3. The system according to claim 1, **characterised in that** coils are used as the pulsed electromagnetic field emitters.

4. The system according to claim 1, **characterised in that** piezoelectric emitters selected from a group including rectangular, disk or ring-shaped ceramic piezoelectrics, sandwich-type batteries made of silver-plated PVDF piezoelectric film, polymer piezoelectric materials are used as the pulsed ultrasonic emitters (3a, 3b).

5. The system according to claim 1 or 4, **characterised in that** ceramic discs are used as the piezoelectric emitters.

6. The system according to claim 1, **characterised in that** as the power supply and battery charging module, lithium-ion batteries are used, connected in series to form packs, which are in turn connected in parallel to form a 2S2P configuration.

7. The system according to claim 1, **characterised in that** the housing (9) of the controller (5) is in the form of a casing in which the electronic circuits of the controller are mounted, enclosed by a front cover (10) with a set of four mounting holes (11) by which the front cover (10) is fixed to the housing, wherein the front cover (10) has a hole (12) for the display and holes (13, 14, 15) for the buttons, for user interaction with the device, in the rear wall of the housing there are catches (16) with retainers (17), allowing the casing to be mounted on a belt, which passes through these retainers, as well as a hole (18) for connecting the battery with the PCB, in the front wall there is a hole (19) for the battery power connector, a hole (20) for the USB programming connector, and in the side wall there is a hole (21) for the connector for connecting therapeutic insoles.

8. A method of electrical stimulation of the diabetic foot by implementing a system comprising therapeutic plantar insoles with pulsed electromagnetic field emitters and pulsed ultrasound emitters, connected by an electrical wiring assembly (4) to a portable controller (5) configured to deliver electrical stimulation to the patient's feet, **characterised in that** it comprises the steps of:
- selecting a therapeutic programme to achieve the desired effect,
- checking, for the selected therapeutic programme, the information provided by the system, which should be taken into account,
- administering electrical stimulation to the patient via the portable controller (5),
- subjecting the patient to alternatingstimulation with pulsed electromagnetic field and pulsed ultrasound with pauses according to the therapeutic programme used,
- adjusting the length and multiplicity of stimulation,
- stopping stimulation if necessary.
